(19) Europäisches Patentamt
European Patent Office
Office européen des brevets

(11) **EP 0 549 438 B1**

(12) **FASCICULE DE BREVET EUROPEEN**

(45) Date de publication et mention
de la délivrance du brevet:
**17.09.1997 Bulletin 1997/38**

(51) Int. Cl.⁶: **A61N 1/08**

(21) Numéro de dépôt: **92403474.7**

(22) Date de dépôt: **21.12.1992**

(54) **Appareil implanté de stimulation, cardioversion et/ou défibrillation cardiaque avec contrôle automatique de la sensibilité**

Apparat zur Herzreizung und/oder Herzenflimmerung mit automatischen Überwachung von Empfindlichkeit

Apparatus to stimulate, cardiovert, and/or defibrillate a heart with automatic sensitivity monitoring

(84) Etats contractants désignés:
**BE CH DE ES FR GB IT LI SE**

(30) Priorité: **23.12.1991 FR 9115980**

(43) Date de publication de la demande:
**30.06.1993 Bulletin 1993/26**

(73) Titulaire: **ELA MEDICAL (Société anonyme)**
**F-92541 Montrouge Cédex (FR)**

(72) Inventeurs:
• **Henry, Christine**
  **F-75014 Paris (FR)**

• **Nitzsche, Rémy**
  **F-78650 Beynes (FR)**

(74) Mandataire: **Laget, Jean-Loup**
**Cabinet Loyer,**
**78, avenue Raymond Poincaré**
**75116 Paris (FR)**

(56) Documents cités:
**EP-A- 0 321 764**　　　**DE-A- 2 604 460**
**US-A- 3 986 496**　　　**US-A- 4 226 245**

## Description

L'invention concerne un procédé de contrôle automatique de la sensibilité pour appareil implanté de stimulation, cardioversion et/ou défibrillation cardiaque, et l'appareil correspondant.

Dans les appareils de contrôle cardiaque, tels que les stimulateurs et les défibrillateurs, les signaux électriques reçus ont des amplitudes réparties dans une gamme s'étendant par exemple de quelques dixièmes de millivolt, (amplitude de l'onde P voisine de 0,4 mV), à quelques millivolts, (amplitude de l'onde R comprise entre 5 et 10mV). Pour assurer un fonctionnement satisfaisant quelle que soit l'amplitude du signal reçu, certains de ces appareils sont munis d'un contrôle automatique de gain.

Le document FR 2.606.644 décrit un dispositif implantable avec fonction de stimulation et de cardioversion, utilisant un contrôle automatique de gain dans le circuit d'amplitude des signaux cardiaques faibles, tels que ceux de la fibrillation ventriculaire.

Le document EP - A - 0 321 764 décrit un détecteur des évènements cardiaques dont l'amplitude dépasse un seuil. A partir de la différence mesurée entre l'amplitude et le seuil, et d'une valeur désirée de seuil, le détecteur commande une nouvelle valeur de seuil.

Le document EP 0 340 045 décrit un appareil de cardioversion utilisant trois sensibilités : la première, moyenne, pour la détection du rythme sinusal et de la tachycardie ventriculaire, la deuxième, plus grande, pour différentier la fibrillation ventriculaire de l'asystolie, et la troisième, plus faible, pour différentier l'onde R et l'onde T lésionnelle à grande amplitude.

Mais l'une de ces trois sensibilités est choisie en fonction de l'état de l'appareil (période de suspicion de tachycardie, ou période post-choc), et elle est maintenue au moins jusqu'au prochain cycle.

Dans un tel cas de sensibilité commutable entre trois valeurs il existe deux types d'inconvénients. Le premier est la sur-détection (ou oversensing) lorsque la sensibilité est très grande : des ondes T peuvent alors être détectées et prises pour des ondes R sur lesquelles le stimulateur se recycle, et peuvent induire en erreur le système d'analyse du rythme (rythme jugé plus rapide qu'il ne l'est en réalité). Le second est la sous-détection (ou undersensing) lorsque la sensibilité est très faible : des ondes R peuvent ne pas être détectées, et le fonctionnement de l'appareil n'est plus adapté au coeur du patient.

Un but de l'invention est de proposer un procédé de contrôle automatique de la sensibilité permettant d'éviter la sur-détection et la sous-détection, en assurant une variation de la sensibilité en cours de cycle cardiaque.

L'invention a pour objet un appareil de stimulation, cardioversion et/ou défibrillation cardiaque, comportant des moyens de détection des complexes ventriculaires, des moyens de mesure de l'amplitude desdits complexes et des moyens de réglage du seuil de sensibilité, caractérisé en ce que : initialement les moyens de réglage du seuil de sensibilité fixent le seuil à une valeur faible (Smax) de façon à avoir une sensibilité maximale, et ensuite lorsque les moyens de détection détectent la présence d'un complexe ventriculaire, les moyens de mesure mesurent l'amplitude dudit complexe pendant la durée d'une fenêtre dans la période réfractaire absolue ventriculaire, et les moyens de réglage du seuil de sensibilité fixent le seuil à l'issue de la période réfractaire absolue ventriculaire à une valeur qui est fonction de l'amplitude du complexe mesuré, et subséquemment, au cours du même cycle cardiaque, lesdits moyens de réglage font décroître par paliers la valeur du seuil de sensibilité vers ladite valeur faible (Smax).

Selon d'autres caractéristiques de l'invention

- la décroissance par paliers du seuil de sensibilité se fait par décréments (DecS) ;

- une période maximale (Tmax) est prévue, comptée à partir de la détection, et à la fin de laquelle le seuil de sensibilité est ramené à ladite valeur faible (Smax) correspondant à la sensibilité maximale.

- l'amplitude (Amp) du complexe ventriculaire est mesurée dans une fenêtre de temps de 16 à 110 ms, de préférence 64 ms, à compter de la détection.

D'autres caractéristiques ressortent de la description qui suit faite avec référence au dessin annexé sur lequel on peut voir :

Figure 1 : un schéma simplifié d'un exemple de signal cardiaque ventriculaire typique filtré et redressé, avec une représentation des seuils de sensibilité dans le cadre du procédé selon l'invention ;

Figure 2 : un schéma simplifié d'un exemple de signal cardiaque correspondant à la survenue d'une fibrillation ventriculaire, avec représentation des seuils de sensibilité dans le cadre du procédé selon l'invention.

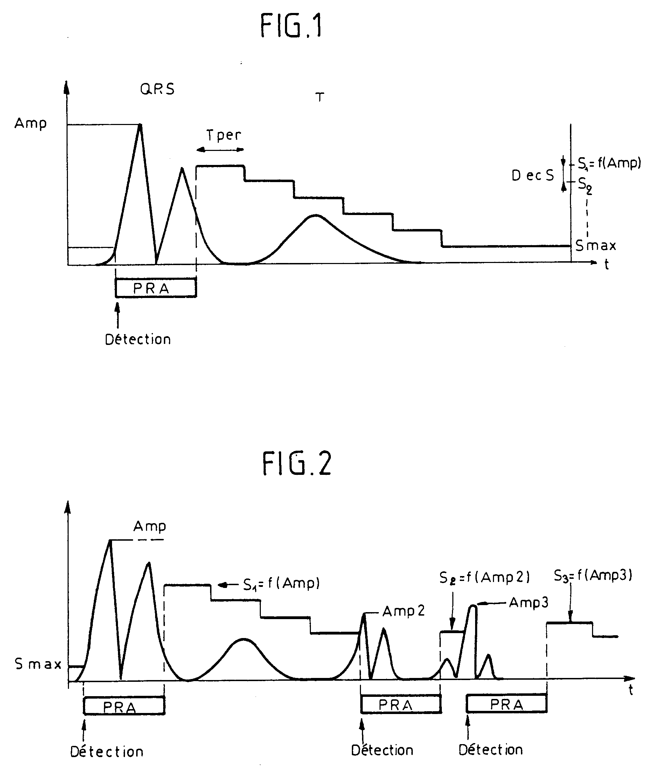

Sur les figures 1 et 2, le temps t est porté en abscisses et l'amplitude A des signaux cardiaques redressés en ordonnées.

Sur la figure 1, les ondes R et T d'un signal cardiaque classique sont représentées. Sont également représentés : le seuil de sensibilité maximale Smax, le pas de décroissance DecS du seuil de sensibilité, à droite de la Figure, l'axe vertical des seuils de sensibilité, et la ligne brisée correspondant aux variations du seuil de sensibilité au cours du cycle cardiaque.

L'amplitude des signaux ventriculaires en fibrillation (Fig. 2) est faible par rapport à l'amplitude des signaux en rythme sinusal : pour pouvoir détecter la fibrillation ventriculaire, il faut donc une grande sensibilité, c'est-à-dire un seuil de sensibilité Smax faible.

Mais si la sensibilité est grande, et le seuil Smax faible, alors l'onde T (Fig. 1) est détectée et risque d'être prise pour une onde R (cas de sur-détection).

Pour éviter la détection de l'onde T, il est possible de prévoir une période réfractaire absolue ventriculaire suffisamment longue (supérieure à 300 ms). Mais, dans un appareil de surveillance cardiaque dont la fonction est de détecter des rythmes à couplage court, (intervalle RR inférieur à 300 ms), cette solution n'est guère envisageable.

Selon l'invention, la sensibilité de l'appareil au cours d'un cycle cardiaque est asservie à l'amplitude du signal ventriculaire sur lequel le cycle a débuté.

Un complexe QRS est détecté si son amplitude est supérieure au seuil de sensibilité au moment de la détection.

En cas de détection d'un événement, deux periodes commencent : d'une part une période réfractaire absolue ventriculaire PRA de 110 ms par exemple, d'autre part une fenêtre de 16 à 110 ms par exemple, de préférence 64 ms. Pendant la durée de la fenêtre, l'amplitude Amp du signal cardiaque redressé est mesurée.

Après la fenêtre, et pendant la PRA, est calculée la valeur S1 du seuil de sensibilité à appliquer à la fin de la PRA. Cette valeur S1 du seuil est une fonction de l'amplitude Amp du signal, par exemple : S1 = Amp x 75%.

La valeur S1 du seuil est rendue effective à la fin de la PRA, pendant une période Tper.

A la fin de la période Tper, la valeur S1 du seuil de sensibilité est diminuée d'un décrément DecS, c'est-à-dire que la sensibilité de l'appareil augmente, et la nouvelle valeur S2 du seuil de sensibilité est appliquée pendant une nouvelle période Tper.

Le processus se poursuit jusqu'à ce que :

- soit la valeur du seuil de sensibilité atteigne la valeur Smax correspondant à la sensibilité maximale,

- soit une nouvelle détection intervienne, qui relance le processus.

De cette manière, dans le cas d'un rythme sinusal (Fig. 1) l'évolution de la sensibilité est telle que l'onde T n'est pas détectée. En effet, la détection de l'onde R entraîne une réduction de la sensibilité qui masque l'onde T. Puis la sensibilité augmente pas-à-pas au cours du cycle, permettant la détection d'événements de faible amplitude.

Sur la figure 2, la partie gauche représente un signal cardiaque sinusal semblable à celui de la Fig. 1, et la partie droite représente le début d'un trouble cardiaque de type fibrillation. L'amplitude des signaux de fibrillation (Amp2, Amp3) est nettement inférieure à celle d'un signal sinusal Amp.

En partant de la gauche sur la Figure 2, le seuil de sensibilité maximal Smax permet de détecter l'onde R et de déclencher la période réfractaire absolue ventriculaire PRA, et la fenêtre de mesure de l'amplitude Amp du signal redressé. A la fin de la PRA est appliquée la valeur S1 du seuil de sensibilité qui est fonction de l'amplitude mesurée : S1 = f (Amp).

Après une période de durée Tper, la valeur du seuil de sensibilité est diminuée d'un décrément DecS.

Après la nouvelle période Tper, la valeur du seuil de sensibilité est diminuée d'un décrément DecS, et ainsi de suite, jusqu'à ce qu'intervienne la détection d'un deuxième signal cardiaque.

Cette détection déclenche une nouvelle PRA et une nouvelle fenêtre pendant laquelle est mesurée l'amplitude Amp2 du signal. S'agissant d'un signal de fibrillation, son amplitude Amp2 est sensiblement inférieure à l'amplitude Amp du signal sinusal. A la fin de la PRA, le nouveau seuil de sensibilité est appliqué : S2 = f (Amp2).

Pendant la période Tper, dans l'exemple représenté, survient une nouvelle détection, qui déclenche une nouvelle PRA et une nouvelle fenêtre pendant laquelle est mesurée l'amplitude Amp3 du nouveau signal cardiaque. A la fin de la PRA est appliqué le seuil de sensibilité S3 = f (Amp3). A la fin d'une nouvelle période Tper, la valeur S3 du seuil est diminuée d'un décrément DecS, et ainsi de suite.

Dans le procédé selon l'invention, le seuil de sensibilité maximale Smax est toujours faible, et la sensibilité correspondante est donc toujours grande, ce qui permet de détecter des événements cardiaques de faible amplitude.

A partir de la détection d'un événement, c'est-à-dire en début de cycle cardiaque, le seuil de sensibilité est calculé en fonction de l'amplitude maximale du signal cardiaque détecté, et donc la sensibilité est diminuée.

Après la période réfractaire absolue PRA, le seuil de sensibilité est diminué par décréments DecS et par paliers de durée Tper, jusqu'à ce qu'il redescende au seuil de sensibilité maximale Smax, ou qu'un nouvel événement soit détecté.

Ainsi, après une détection, la sensibilité devient faible, et elle augmente par paliers au cours du déroulement du cycle en tendant vers sa valeur maximale.

En conséquence, le phénomène de sur-détection est évité car l'onde T a une amplitude inférieure au seuil de sensibilité à l'instant correspondant au maximum de l'onde T; et le phénomène de sous-détection est évité aussi, car le seuil de sensibilité maximale est faible en vue de la détection des ondes R d'amplitude faible (cas de la fibrillation).

Dans le cadre de l'invention peuvent être prévus : un seuil de sensibilité minimale Smin et une période maximale Tmax, comptée à partir de la détection, et à la fin de laquelle le seuil de sensibilité redescend impérativement à la valeur Smax.

Les paramètres Smax, Smin, DecS, Tper et Tmax sont programmables.

La détection des complexes ventriculaires et la mesure de l'amplitude de ces complexes détectés sont assurées par des moyens électroniques classiques ; la variation de la sensibilité au cours du cycle cardiaque est contrôlée par un logiciel.

Le déroulement des opérations logiques est le suivant sur la base d'une détection intervenue au temps t = 0 :

. initialisation du contrôle automatique de sensibilité,

- pendant la PRA :

. mesure de l'amplitude maximale Amp du complexe ventriculaire dans la fenêtre de 64 ms,

. Si PRA < Tmax :

. calcul du seuil de sensibilité S1 à positionner en fin de PRA

S1 = Amp x 75%,

. si S1 > Smax, on garde la valeur calculée,

. sinon on fixe S1 = Smax

. Si PRA >= Tmax, alors S1 = Smax

- de t = PRA , par exemple t = 110 ms, à T = Tmax , pendant chaque période Tper :

. la valeur du seuil de sensibilité calculée pendant la période précédente est utilisée et maintenue par des moyens électroniques classiques,

. calcul de la valeur du seuil de sensibilité à positionner à la fin de la période Tper en cours,

. S(t + Tper) = S(t) - DecS

en vérifiant que (t + Tper) est inférieure à Tmax et que la valeur du seuil de sensibilité calculée est supérieure à Smax. Si l'une des deux conditions ne se vérifie pas, alors S(t + Tper) = Smax .

Dans le cas d'une stimulation ventriculaire, le déroulement des opérations logiques est identique à l'exception de l'initialisation pour laquelle il n'y a pas de mesure d'amplitude à effectuer : le seuil de sensibilité est fixé à : S1 = Smin - DecS .

Dans le cas d'un stimulateur cardiaque, les paramètres peuvent être programmés pour faire un réglage de la sensibilité en vue d'améliorer le fonctionnement du stimulateur : on évite la surdétection, et avec une PRA courte, on a une meilleure écoute de la chambre ventriculaire.

Dans le cas d'un défibrillateur, le procédé de contrôle automatique de la sensibilité permet de détecter les ondes R rapides et éventuellement de faible amplitude correspondant à la tachycardie ventriculaire, sans détecter les ondes T, et il permet ainsi d'identifier une tachycardie ventriculaire.

**Revendications**

1. Appareil de stimulation, cardioversion et/ou défibrillation cardiaque, comportant des moyens de détection des complexes ventriculaires, des moyens de mesure de l'amplitude desdits complexes et des moyens de réglage du seuil de sensibilité, caractérisé en ce que : initialement les moyens de réglage du seuil de sensibilité fixent le seuil à une valeur faible (Smax) de façon à avoir une sensibilité maximale, et ensuite lorsque les moyens de détection détectent la présence d'un complexe ventriculaire, les moyens de mesure mesurent l'amplitude dudit complexe pendant la durée d'une fenêtre dans la période réfractaire absolue ventriculaire, et les moyens de réglage du seuil de sensibilité fixent le seuil à l'issue de la période réfractaire absolue ventriculaire à une valeur qui est fonction de l'amplitude du complexe mesuré, et subséquemment, au cours du même cycle cardiaque, lesdits moyens de réglage font décroître par paliers la valeur du seuil de sensibilité vers ladite valeur faible (Smax).

2. Appareil de stimulation, cardioversion et/ou défibrillation cardiaque selon la revendication 1, caractérisé en ce que la décroissance par paliers du seuil de sensibilité se fait par décréments (DecS).

3. Appareil de stimulation, cardioversion et/ou défibrillation cardiaque selon la revendication 1, caractérisé en ce qu'une période maximale (Tmax) est prévue, comptée à partir de la détection, et à la fin de laquelle le seuil de sensibilité est ramené à ladite valeur faible (Smax) correspondant à la sensibilité maximale.

4. Appareil de stimulation, cardioversion et/ou défibrillation cardiaque selon la revendication 1, caractérisé en ce que l'amplitude (Amp) du complexe ventriculaire est mesurée dans une fenêtre de temps de 16 à 110 ms, de préférence 64 ms à compter de la détection.

**Claims**

1. Apparatus for cardiac stimulation, cardioversion and/or defibrillation, comprising means for detecting ventricular complexes, means for measuring the amplitude of said complexes, and means for adjusting sensitivity threshold, characterized in that : initially the means for adjusting sensitivity threshold set the threshold at a low value (Smax) so as to have maximum sensitivity, and thereafter, upon detection of a ventricular complex by said detection means, said measuring means measure the amplitude of said complex within duration of a window in the ventricular absolute refractory period, and means for adjusting sensitivity threshold set the threshold, at the end of said ventricular absolute refractory period, to a value which is a function of amplitude of the measured complex, and subsequently , during same cardiac cycle, said adjusting means make the sensitivity threshold value decrease in stages towards said low value (Smax).

2. Apparatus for cardiac stimulation, cardioversion and/or defibrillation according to claim 1, characterized in that the decreasing in stages of sensitivity threshold is made by decrements (DecS).

3. Apparatus for cardiac stimulation, cardioversion and/or defibrillation according to claim 1, characterized in that a maximum period (Tmax) is provided, counted from detection, and at the end of which the sensitivity threshold is returned to said low value (Smax) corresponding to maximum sensitivity.

4. Apparatus for cardiac stimulation, cardioversion and/or defibrillation according to claim 1 characterized in that the amplitude (Amp) of the ventricular complex is measured within a time window of 16 to 100 ms, preferably 64 ms counted from the detection.

**Patentansprüche**

1. Vorrichtung zur Stimulation, Kardioversion und/oder Defibrillation eines Herzes, mit

Einrichtungen zur Erfassung von Kammerkomplexen,
Einrichtungen zur Messung der Amplitude der Kammerkomplexe und
Einrichtungen zur Regelung einer Empfindlichkeitsschwelle,
**dadurch gekennzeichnet, daß**
anfänglich die Einrichtungen zur Regelung der Empfindlichkeitsschwelle die Schwelle auf einen niedrigen Wert (Smax) festlegen, um eine maximale Empfindlichkeit zu erhalten, und dann die Einrichtungen zur Messung die Amplitude des Kammerkomplexes während der Dauer eines Zeitfensters innerhalb der absoluten ventrikulären Refraktärphase messen, sobald die Einrichtungen zur Erfassung das Vorliegen eines Kammerkomplexes erfassen und
die Einrichtungen zur Regelung der Empfindlichkeitsschwelle die Schwelle am Ende der absoluten ventrikulären Refraktärphase auf einen Wert festlegen, der von der gemessenen Amplitude des Kammerkomplexes abhängig ist, und
anschließend die Einrichtungen zur Regelung den Wert der Empfindlichkeitsschwelle, im Verlauf desselben Herzzyklus, abschnittsweise auf den niedrigen Wert (Smax) verringern.

2. Vorrichtung zur Stimulation, Kardioversion und/oder Defibrillation eines Herzes nach Anspruch 1,
**dadurch gekennzeichnet, daß**
die abschnittsweise Verringerung der Empfindlichkeitsschwelle in Dekrementen (DecS) erfolgt.

3. Vorrichtung zur Stimulation, Kardioversion und/oder Defibrillation eines Herzes nach Anspruch 1,
**dadurch gekennzeichnet, daß**
ab der Erfassung ein maximaler Zeitraum (Tmax) vorgesehen ist, an dessen Ende die Empfindlichkeitsschwelle auf den der maximalen Empfindlichkeit entsprechenden niedrigen Wert (Smax) zurückgeführt wird.

4. Vorrichtung zur Stimulation, Kardioversion und/oder Defibrillation eines Herzes nach Anspruch 1,
**dadurch gekennzeichnet, daß**
die Amplitude (Amp) des Kammerkomplexes während eines Zeitfensters von 16 bis 110 ms, vorzugsweise von 64 ms, ab der Erfassung gemessen wird.

# FIG.1

# FIG.2

6